(19) **Europäisches Patentamt / European Patent Office / Office européen des brevets**

(11) **EP 4 640 816 A1**

(12) **EUROPEAN PATENT APPLICATION**
published in accordance with Art. 153(4) EPC

(43) Date of publication:
**29.10.2025 Bulletin 2025/44**

(21) Application number: **23919919.3**

(22) Date of filing: **06.12.2023**

(51) International Patent Classification (IPC):
**C12M 1/34** (2006.01)    **G06T 7/00** (2017.01)

(52) Cooperative Patent Classification (CPC):
**C12M 1/34; G06T 7/00**

(86) International application number:
**PCT/JP2023/043687**

(87) International publication number:
**WO 2024/161784 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.01.2023 JP 2023012251**

(71) Applicant: **FUJIFILM Corporation**
**Tokyo 106-8620 (JP)**

(72) Inventors:
• **INOUE, Tsutomu**
**Ashigarakami-gun, Kanagawa 258-8577 (JP)**
• **TOMINAGA, Shunsuke**
**Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **DETERMINATION ASSISTANCE DEVICE, METHOD FOR OPERATING DETERMINATION ASSISTANCE DEVICE, AND OPERATION PROGRAM FOR DETERMINATION ASSISTANCE DEVICE**

(57)    A determination support device that supports determination of monoclonality of a cell seeded in a container includes a processor, in which the processor is configured to acquire a same-day captured image, which is a captured image of the container on the day the cell is seeded, and a next-day captured image, which is a captured image of the container on the day after the cell is seeded, extract a cell-like object, which is any of the cell or a similar object that is morphologically similar to the cell, from each of the same-day captured image and the next-day captured image, evaluate similarity between a first cell-like object, which is the cell-like object extracted from the same-day captured image, and a second cell-like object, which is the cell-like object extracted from the next-day captured image and corresponds in position to the first cell-like object, and display the first cell-like object of which the similarity is relatively low in an identifiable manner.

FIG. 14

EP 4 640 816 A1

**Description**

BACKGROUND OF THE INVENTION

1. Field of the Invention

The technology of the present disclosure relates to a determination support device, an operation method of a determination support device, and an operation program of a determination support device.

2. Description of the Related Art

Recently, there has been an active production of antibody pharmaceuticals by culturing Chinese hamster ovary (hereinafter, referred to as CHO) cells into which an antibody gene is incorporated and causing the CHO cells to produce an antibody. The CHO cells are seeded and cultured, for example, one by one in each of a plurality of wells of a well plate. Then, among the CHO cells in the respective wells, a CHO cell having an excellent antibody production ability (referred to as a stable expression cell line) is selected. In this case, regulatory authorities such as the United States Food and Drug Administration (US FDA) require assurance that one CHO cell is seeded in the well without mistake and that the antibody is produced from one CHO cell without mistake (cellular monoclonality, also referred to as monoclonality). Therefore, for example, as in JP2022-509201A, a technology for supporting determination of the cellular monoclonality has been proposed.

SUMMARY OF THE INVENTION

A captured image of a well on the day cell is seeded (hereinafter, referred to as a same-day captured image) is used for the determination of the cellular monoclonality. Note that, in a case in which the determination is made only based on the same-day captured image, the following problem arises. That is, there may be a CHO cell that floats in a culture solution without being sedimented on a bottom surface of the well on the day the cell is seeded, and such a CHO cell is not shown in the same-day captured image, or even in a case in which the CHO cell is shown, the CHO cell is shown in a manner that is far from the manner in which the typical CHO cell is shown after being sedimented on the bottom surface of the well. For this reason, there is a risk that it is determined that no CHO cell is present even though a floating CHO cell is actually present.

As a method for solving such a problem, a method of extracting a CHO cell-like object, which is any of a CHO cell or a similar object that is morphologically similar to the CHO cell, such as a floating CHO cell, from the same-day captured image and allowing a user to thoroughly check the extracted CHO cell-like object can be considered. However, since one well plate has, for example, 96 wells and a large number of well plates, for example, 10 to 100 well plates are also used, the above method requires a large amount of time and effort for check by the user.

One embodiment according to the technology of the present disclosure provides a determination support device, an operation method of a determination support device, and an operation program of a determination support device capable of reducing time and effort for determining the monoclonality of a cell seeded in a container.

According to the present disclosure, there is provided a determination support device that supports determination of monoclonality of a cell seeded in a container, the determination support device comprising: a processor, in which the processor is configured to acquire a same-day captured image, which is a captured image of the container on the day the cell is seeded, and a next-day captured image, which is a captured image of the container on the day after the cell is seeded, extract a cell-like object, which is any of the cell or a similar object that is morphologically similar to the cell, from each of the same-day captured image and the next-day captured image, evaluate similarity between a first cell-like object, which is the cell-like object extracted from the same-day captured image, and a second cell-like object, which is the cell-like object extracted from the next-day captured image and corresponds in position to the first cell-like object, and display the first cell-like object of which the similarity is relatively low in an identifiable manner.

It is preferable that the processor is configured to display the first cell-like object and the second cell-like object that corresponds in position to the first cell-like object side by side.

It is preferable that the processor is configured to display a first indicator indicating a position of the first cell-like object in the same-day captured image, and display a second indicator indicating a position of the second cell-like object in the next-day captured image.

It is preferable that the processor is configured to extract the cell-like object using a machine learning model that distinguishes between a plurality of classes including the cell and the similar object, and change a display form of the first cell-like object according to a probability of the cell calculated by the machine learning model.

It is preferable that the cell is a Chinese hamster ovary cell into which an antibody gene is incorporated.

According to the present disclosure, there is provided an operation method of a determination support device that supports determination of monoclonality of a cell seeded in a container, the operation method comprising: acquiring a same-day captured image, which is a captured image of the container on the day the cell is seeded, and a next-day captured image, which is a captured image of the container on the day after the cell is seeded; extracting a cell-like object, which is any of the cell or a similar object that is morphologically similar to the cell, from each of the same-day captured image and the next-day captured image; evaluating similarity between a first cell-like object, which is the cell-like object extracted from the same-day captured image, and a second cell-like object, which is the cell-like object extracted from the next-day captured image and corresponds in position to the first cell-like object; and displaying the first cell-like object of which the similarity is relatively low in an identifiable manner.

According to the present disclosure, there is provided an operation program of a determination support device that supports determination of monoclonality of a cell seeded in a container, the operation program causing a computer to execute a process comprising: acquiring a same-day captured image, which is a captured image of the container on the day the cell is seeded, and a next-day captured image, which is a captured image of the container on the day after the cell is seeded; extracting a cell-like object, which is any of the cell or a similar object that is morphologically similar to the cell, from each of the same-day captured image and the next-day captured image; evaluating similarity between a first cell-like object, which is the cell-like object extracted from the same-day captured image, and a second cell-like object, which is the cell-like object extracted from the next-day captured image and corresponds in position to the first cell-like object; and displaying the first cell-like object of which the similarity is relatively low in an identifiable manner.

According to the technology of the present disclosure, it is possible to provide a determination support device, an operation method of a determination support device, and an operation program of a determination support device capable of reducing time and effort for determining the monoclonality of a cell seeded in a container.

BRIEF DESCRIPTION OF THE DRAWINGS

Fig. 1 is a diagram showing a state in which CHO cells are seeded in wells of a well plate and the wells are imaged by an imaging device, and a determination support device.
Fig. 2 is a diagram showing a state in which a same-day captured image group, which is a set of same-day captured images that are captured images of wells on the day CHO cells are seeded, is transmitted from an imaging device to a determination support device.
Fig. 3 is a diagram showing a state in which a next-day captured image group, which is a set of next-day captured images that are captured images of wells on the day after CHO cells are seeded, is transmitted from the imaging device to the determination support device.
Fig. 4 is a block diagram of a computer that constitutes the determination support device.

Fig. 5 is a block diagram showing a processing unit of a CPU of the determination support device.
Fig. 6 is a diagram showing a detailed configuration of an extraction model.
Fig. 7 is a diagram showing processing of inputting a same-day captured image to the extraction model and outputting a same-day extraction result of the day from the extraction model.
Fig. 8 is a diagram showing processing of inputting a next-day captured image to the extraction model and outputting a next-day extraction result of the day from the extraction model.
Fig. 9 is a diagram showing a same-day extraction result group.
Fig. 10 is a diagram showing a next-day extraction result group.
Fig. 11 is a diagram showing a set of a first cell-like object and a second cell-like object for which similarity is evaluated by an evaluation unit.
Fig. 12 is a diagram showing a detailed configuration of the evaluation unit.
Fig. 13 is a diagram showing an evaluation result group.
Fig. 14 is a diagram showing an evaluation result display screen.
Fig. 15 is a diagram showing a comparison display screen.
Fig. 16 is a flowchart showing a processing procedure of the determination support device.
Fig. 17 is a flowchart showing a processing procedure of the determination support device.
Fig. 18 is a flowchart showing a processing procedure of the determination support device.
Fig. 19 is a diagram showing another example of the evaluation result display screen.
Fig. 20 is a diagram showing an example of changing a display form of the first cell-like object according to a probability of the cell calculated by the extraction model.
Fig. 21 is a diagram showing another example of changing a display form of the first cell-like object according to a probability of the cell calculated by the extraction model.

DESCRIPTION OF THE PREFERRED EMBODIMENTS

[First Embodiment]

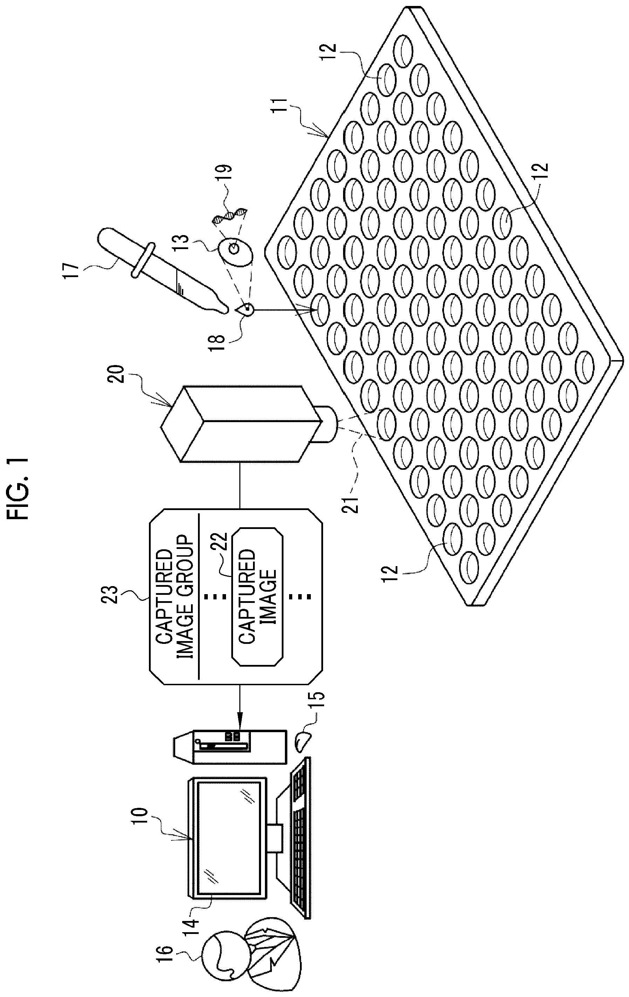

As shown in Fig. 1 as an example, a determination support device 10 according to the technology of the present disclosure supports the determination of the cellular monoclonality of a CHO cell 13 seeded in a well 12 of a well plate 11. The determination support device 10 is, for example, a desktop personal computer and comprises a display 14 that displays various screens and an input device 15 such as a keyboard, a mouse, a touch panel, and/or a microphone for voice input. The determination support device 10 is installed in, for example, a development facility for antibody pharmaceuticals and is operated by a user 16 who is involved in the development of the antibody pharmaceuticals in the development facility.

A plurality of the wells 12 are formed in the well plate 11. Fig. 1 illustrates a so-called 96-well plate in which $12 \times 8 = 96$ wells 12 are formed. A liquid droplet 18 is dispensed into each well 12 by a pipette 17. The liquid droplet 18 contains a CHO cell 13. An antibody gene 19 is incorporated into the CHO cell 13, and the CHO cell 13 produces an antibody that is a source of antibody pharmaceuticals during a culture process. The well 12 is an example of a "container" according to the technology of the present disclosure. In addition, the CHO cell 13 is an example of a "cell" according to the technology of the present disclosure. Although only one well plate 11 is depicted in Fig. 1, in reality, a large number of well plates 11, for example 10 to 100, are used.

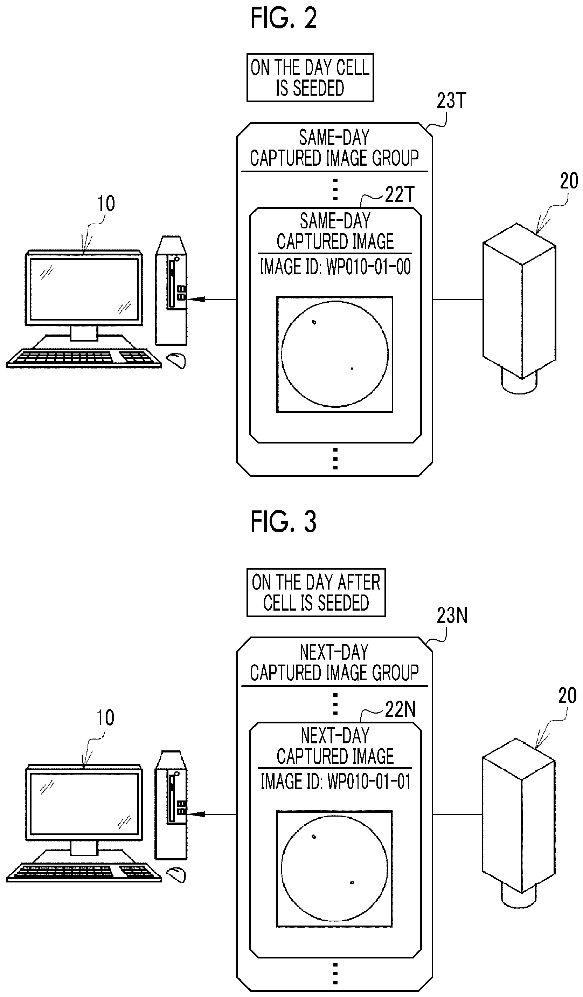

Each well 12 is imaged by an imaging device 20. The imaging device 20 is, for example, a digital phase contrast microscope. The digital phase contrast microscope includes a light source, an optical system, an imaging element, and the like. The light source irradiates the well 12 with illumination light 21. The optical system is composed of a plurality of lenses and the like that capture an optical image of the well 12. The imaging element captures an optical image of the well 12 formed by the optical system and outputs a captured image 22 of the well 12. Image identification data (ID) for identifying each captured image is added to the captured image 22 (see Figs. 2 and 3). In Fig. 1, it is depicted that one well 12 is imaged in its entirety at once, in reality, one well 12 is divided into a number of regions, each of which is imaged, and images obtained for each region are then combined to generate a captured image 22 in which the entirety of one well 12 is shown.

The imaging device 20 has a function of adjusting the focus of the optical system according to a type of the well plate 11 and a scan result of distortion of a bottom surface of the well 12. Therefore, the captured image 22 is a clear image that is focused on the bottom surface of the well 12 and that has no blurriness. The imaging device 20 transmits a captured image group 23, which is a set of the captured images 22 of the respective wells 12, to the determination support device 10.

As shown in Fig. 2 as an example, the imaging device 20 captures the captured image 22 of the well 12 immediately after (on the same day) the CHO cell 13 is seeded. Hereinafter, the captured image 22 will be referred to as a same-day captured image 22T. The imaging device 20 transmits a same-day captured image group 23T, which is a set of the same-day captured images 22T of the respective wells 12, to the determination support device 10.

In addition, as shown in Fig. 3 as an example, the imaging device 20 captures the captured image 22 of the well 12 on the day after the CHO cell 13 is seeded.

Hereinafter, the captured image 22 will be referred to as a next-day captured image 22N. The imaging device 20 transmits a next-day captured image group 23N, which is a set of the next-day captured images 22N of the respective wells 12, to the determination support device 10. As described above, the captured image group 23 includes the same-day captured image group 23T and the next-day captured image group 23N. Although not shown, the imaging device 20 captures the captured image 22 of the well 12 even before the CHO cell 13 is seeded, two days after the CHO cell 13 is seeded, three days after the CHO cell 13 is seeded, and the like.

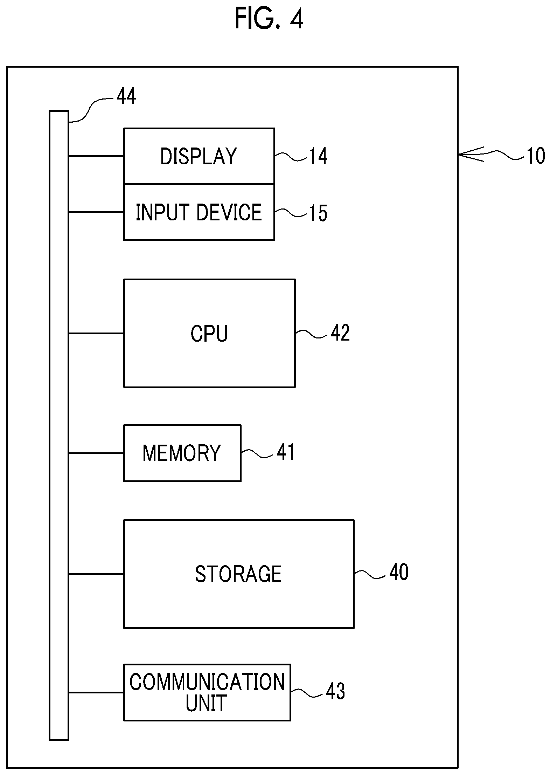

As shown in Fig. 4 as an example, a computer constituting the determination support device 10 comprises a storage 40, a memory 41, a central processing unit (CPU) 42, and a communication unit 43 in addition to the display 14 and the input device 15 described above. These are interconnected via a busline 44.

The storage 40 is a hard disk drive that is built into the computer constituting the determination support device 10 or that is connected via a cable or a network. Alternatively, the storage 40 is a disk array in which a plurality of hard disk drives are connected in series. The storage 40 stores a control program such as an operating system, various application programs, various data associated with these programs, and the like. A solid state drive may be used instead of the hard disk drive.

The memory 41 is a work memory for the CPU 42 to execute processing. The CPU 42 loads the program stored in the storage 40 into the memory 41 and executes processing corresponding to the program. Thus, the CPU 42 collectively controls the respective units of the computer. The CPU 42 is an example of a "processor" according to the technology of the present disclosure. The memory 41 may be built into the CPU 42. The communication unit 43 controls the transmission of various types of information to an external device such as the imaging device 20.

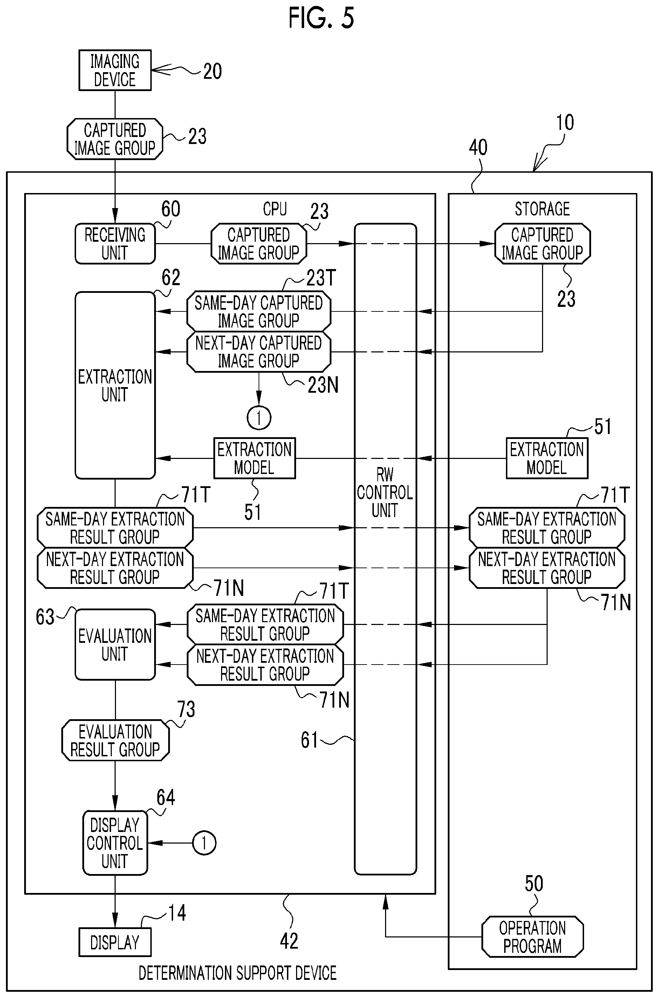

As shown in Fig. 5 as an example, an operation program 50 is stored in the storage 40 of the determination support device 10. The operation program 50 is an application program for causing the computer to function as the determination support device 10. That is, the operation program 50 is an example of "an operation program of a determination support device" according to the technology of the present disclosure. The storage 40 also stores an extraction model 51 and the like. The extraction model 51 is an example of a "machine learning model" according to the technology of the present disclosure.

In a case in which the operation program 50 is activated, the CPU 42 of the computer constituting the determination support device 10 functions as a receiving unit 60, a read/write (hereinafter, referred to as RW) control unit 61, an extraction unit 62, an evaluation unit 63, and a display control unit 64 in cooperation with the memory 41 and the like.

The receiving unit 60 receives the captured image group 23 from the imaging device 20. The receiving unit 60 outputs the received captured image group 23 to the RW control unit 61.

The RW control unit 61 controls the read-out of various data stored in the storage 40 and the storage of various data in the storage 40. For example, the RW control unit 61 stores the captured image group 23 from the receiving unit 60 in the storage 40.

The RW control unit 61 reads out the same-day captured image group 23T and the next-day captured image group 23N, which are included in the captured image group 23, and the extraction model 51 from the storage 40, and outputs the same-day captured image group 23T, the next-day captured image group 23N, and the extraction model 51 that have been read out to the extraction unit 62. In addition, the RW control unit 61 outputs the read-out same-day captured image group 23T and next-day captured image group 23N to the display control unit 64.

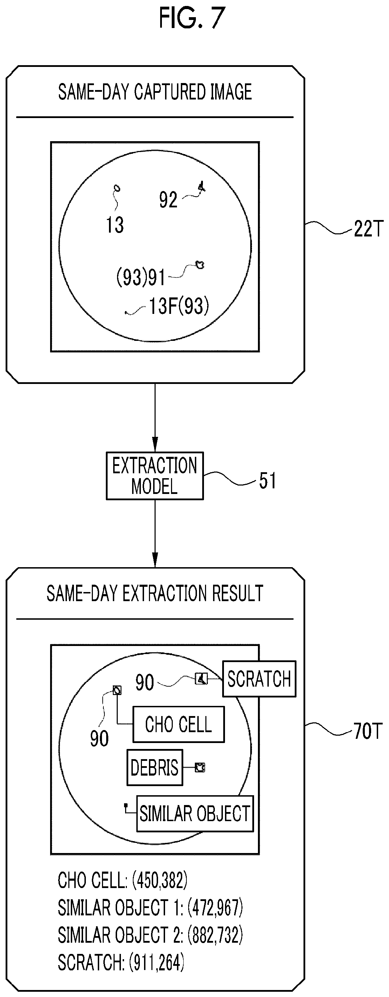

The extraction unit 62 uses the extraction model 51 to extract various objects shown in the same-day captured image 22T of the same-day captured image group 23T and the next-day captured image 22N of the next-day captured image group 23N. The object includes a cell-like object and a non-cell-like object. The cell-like object is any of the CHO cell 13 itself or a similar object 93 (see Fig. 7) that is morphologically similar to the CHO cell 13. Although it cannot be clearly said that the similar object 93 is the CHO cell 13, the similar object 93 is an object whose probability of being the CHO cell 13 is higher than that of the non-cell-like object. The similar object 93 includes the CHO cell 13 that floats in the culture solution without being sedimented on the bottom surface of the well 12 (hereinafter, referred to as a floating CHO cell 13F). In addition, the similar object 93 also includes debris 91 (see Fig. 7) such as dust and dirt. The non-cell-like object is an object that can be clearly distinguished from the CHO cell 13. The non-cell-like object includes a scratch 92 (see Fig. 7) formed on the bottom surface of the well 12.

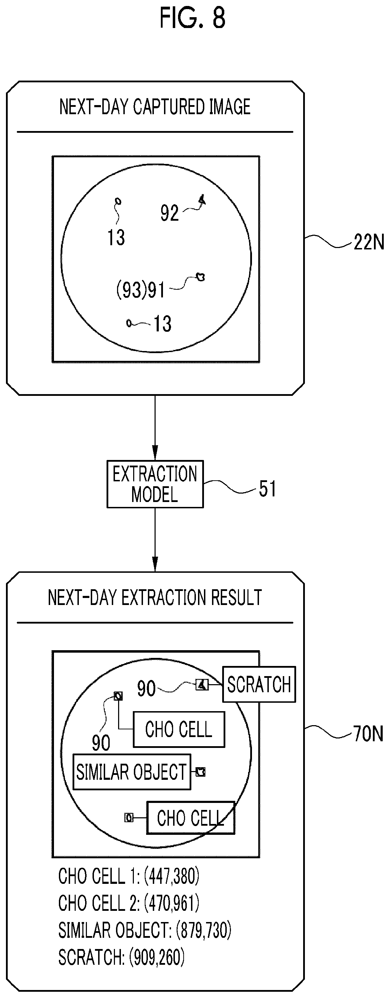

The extraction unit 62 causes the extraction model 51 to output a same-day extraction result 70T (see Fig. 7), which is an extraction result of the object in the same-day captured image 22T. In addition, the extraction unit 62 causes the extraction model 51 to output a next-day extraction result 70N (see Fig. 8), which is an extraction result of the object in the next-day captured image 22N. The extraction unit 62 outputs, to the RW control unit 61, a same-day extraction result group 71T, which is a set of a plurality of the same-day extraction results 70T based on a plurality of the same-day captured images 22T, and a next-day extraction result group 71N, which is a set of a plurality of the next-day extraction results 70N based on a plurality of the next-day captured images 22N.

The RW control unit 61 stores the same-day extraction result group 71T and the next-day extraction result group 71N from the extraction unit 62 in the storage 40. In addition, the RW control unit 61 reads out the same-day extraction result group 71T and the next-day extraction result group 71N from the storage 40, and outputs the read-out same-day extraction result group 71T and next-day extraction result group 71N to the evaluation unit 63.

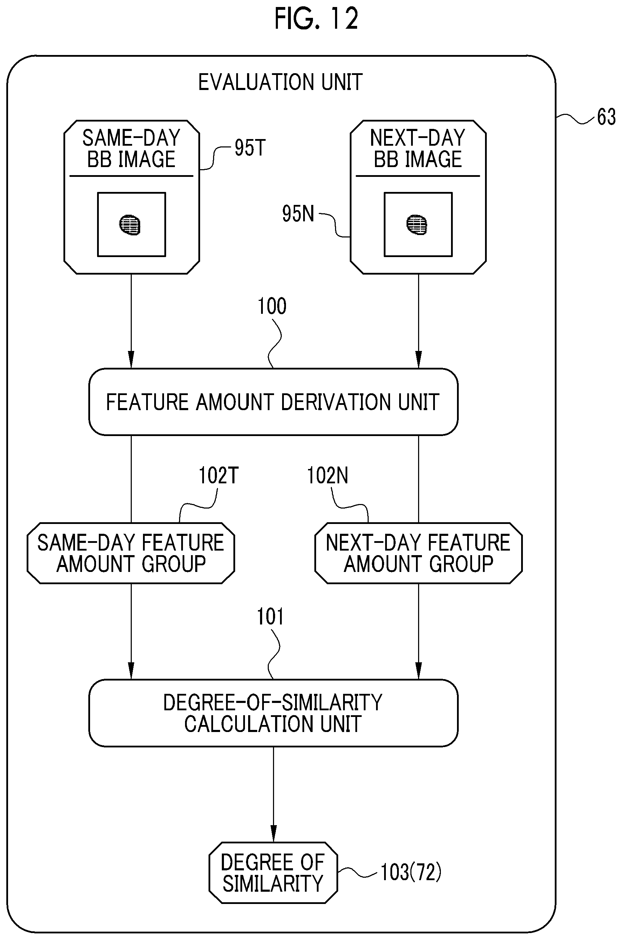

The evaluation unit 63 evaluates similarity between a first cell-like object and a second cell-like object. The first cell-like object is a cell-like object extracted from the same-day captured image 22T in the extraction unit 62. The second cell-like object is a cell-like object extracted from the next-day captured image 22N in the extraction unit 62. The evaluation unit 63 outputs an evaluation result group 73, which is a set of evaluation results 72 (see Fig. 12) of the similarity, to the display control unit 64.

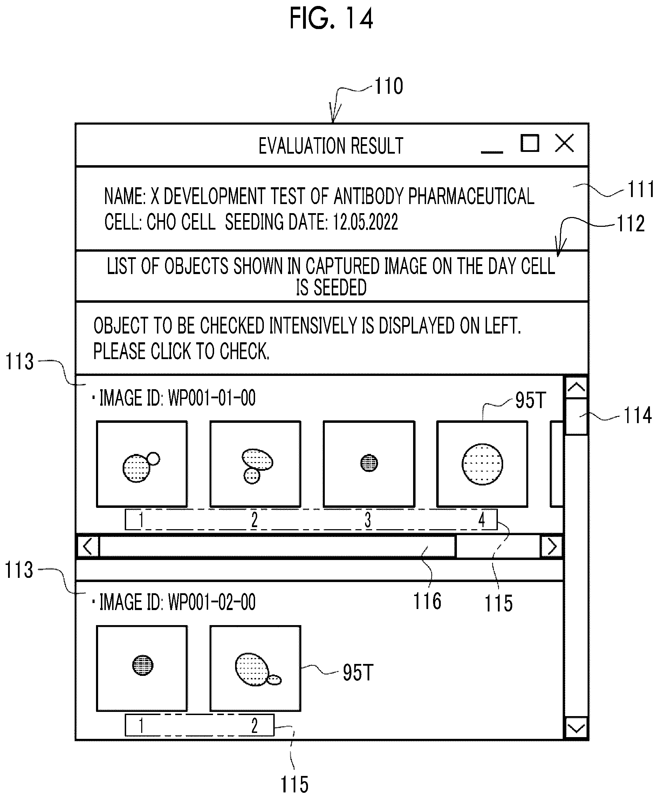

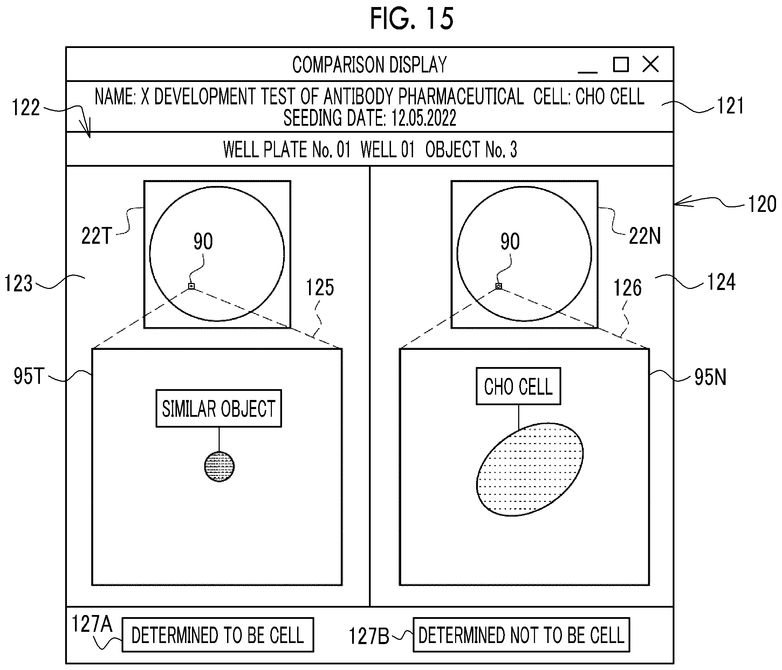

The display control unit 64 performs control of displaying various screens based on the same-day captured image 22T, the next-day captured image 22N, and the evaluation result 72 on the display 14. The various screens include an evaluation result display screen 110 (see Fig. 14), a comparison display screen 120 (see Fig. 15), and the like. The display control unit 64 displays the first cell-like object on the evaluation result display screen 110 according to the evaluation result 72.

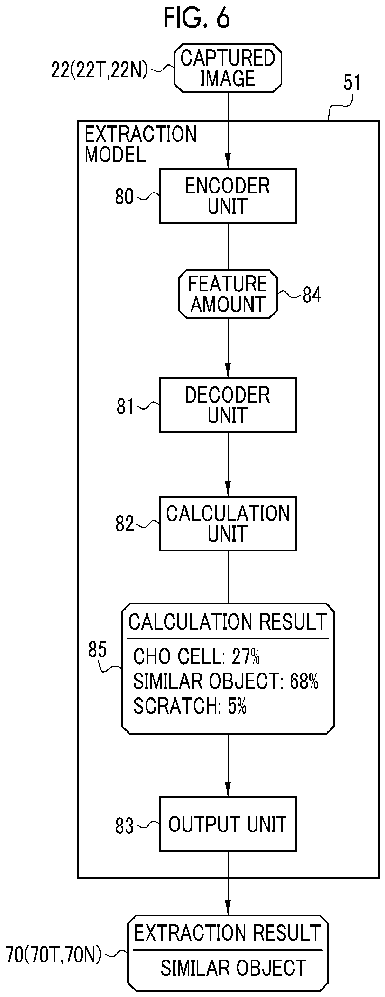

As shown in Fig. 6 as an example, the extraction model 51 includes an encoder unit 80, a decoder unit 81, a calculation unit 82, and an output unit 83. The captured image 22 is input to the encoder unit 80. More specifically, any of the same-day captured image 22T or the next-day captured image 22N is input to the encoder unit 80. The encoder unit 80 converts the input captured image 22 into a feature amount 84. The encoder unit 80 delivers the feature amount 84 to the decoder unit 81. The decoder unit 81 decodes the feature amount 84.

As is well known, the encoder unit 80 includes a convolutional layer that performs convolution processing using a filter, a pooling layer that performs pooling processing such as maximum value pooling, and the like. The same applies to the decoder unit 81. That is, the extraction model 51 is a convolutional neural network (CNN). The encoder unit 80 derives the feature amount 84 by repeating convolution processing using the convolutional layer and pooling processing using the pooling layer on the input captured image 22 a plurality of times. The feature amount 84 represents a feature of a shape and a texture of various objects shown in the captured image 22. The feature amount 84 is a set of a plurality of numerical values. That is, the feature amount 84 is multidimensional data. The number of dimensions of the feature amount 84 is, for example, 512, 1024, or 2048.

The calculation unit 82 calculates the probability based on data generated by decoding the feature amount 84 using the decoder unit 81, and outputs a calculation result 85. The calculation result 85 is used for distinguishing a class of the object shown in the captured image 22 by the output unit 83. The classes in this example are the CHO cell 13, the similar object 93, and the scratch 92. Therefore, the calculation result 85 includes the probability that the object shown in the captured image 22 is the CHO cell 13, the probability that the object is the similar object 93, and the probability that the object is the scratch 92. The sum of these probabilities is 100%. The calculation unit 82 outputs the calculation result 85 to the output unit 83.

The output unit 83 outputs the extraction result 70 corresponding to the calculation result 85. The output unit 83 distinguishes a class having a maximum value among a plurality of the probabilities included in the calculation result 85 as the class of the object shown in the captured image 22. The output unit 83 outputs the extraction result 70 including the distinguished class. The extraction result 70 is the same-day extraction result 70T in a case in which the same-day captured image 22T is input to the encoder unit 80, and is the next-day extraction result 70N in a case in which the next-day captured image 22N is input to the encoder unit 80. Fig. 6 illustrates a case in which the object shown in the captured image 22 is distinguished as the similar object 93 in a case in which the probability of being the CHO cell 13 is 27%, the probability of being the similar object 93 is 68%, and the probability of being the scratch 92 is 5%. In Fig. 6, for convenience of description, only one calculation result 85 is drawn, but, in a case in which there are a plurality of objects shown in the captured image 22, the calculation unit 82 calculates the probability for each of the plurality of objects and outputs those calculation results 85. Therefore, in a case in which there are a plurality of objects shown in the captured image 22, the extraction result 70 including the classes for the plurality of objects is output.

Fig. 7 shows processing in which the extraction unit 62 inputs the same-day captured image 22T to the extraction model 51 and causes the extraction model 51 to output the same-day extraction result 70T. In addition, Fig. 8 shows processing of inputting the next-day captured image 22N to the extraction model 51 and causing the extraction model 51 to output the next-day extraction result 70N. As shown in Figs. 7 and 8, a bounding box 90, which is a square-shaped frame surrounding the extracted object, is added to the same-day extraction result 70T and the next-day extraction result 70N in addition to the distinguished class of the object. In addition, the position of the object is included in the same-day extraction result 70T and the next-day extraction result 70N. The position is, for example, a center position of the bounding box 90, and is represented by XY coordinates with an upper left end of the captured image 22 as an origin. For convenience of description, the object is drawn in the same-day extraction result 70T and the next-day extraction result 70N, but the actual same-day extraction result 70T and next-day extraction result 70N only have information on the position and size of the bounding box 90 and the class.

The same-day captured image 22T shown in Fig. 7 shows the CHO cell 13, the floating CHO cell 13F, the debris 91, and the scratch 92. Therefore, the same-day extraction result 70T shown in Fig. 7 is data including the bounding boxes 90 surrounding the CHO cell 13, the floating CHO cell 13F and the debris 91, which are the similar objects 93, and the scratch 92, as well as the classes "CHO cell," "similar object," and "scratch" added respectively.

The next-day captured image 22N shown in Fig. 8 is an image of the same well 12 as the same-day captured image 22T shown in Fig. 7. The next-day captured image 22N shown in Fig. 8 is the same as the same-day captured image 22T shown in Fig. 7 except that the floating CHO cell 13F is replaced with the CHO cell 13. Therefore, the next-day extraction result 70N shown in Fig. 8 is data including the bounding boxes 90 surrounding two CHO cells 13, the debris 91, which is the similar object 93, and the scratch 92, as well as the classes "CHO cell," "similar object," and "scratch" annotated respectively. The reason why the positions of the objects are slightly different between the same-day extraction result 70T and the next-day extraction result 70N is that the origin of the imaging position by the imaging device 20 is slightly shifted between the day of seeding and the day after seeding due to a mechanical error or the like.

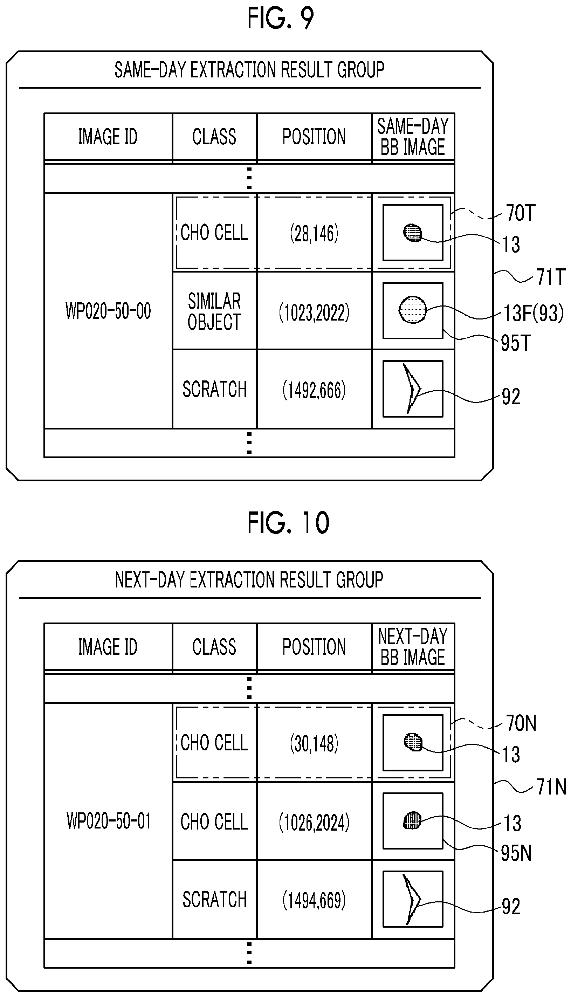

As shown in Fig. 9 as an example, the same-day extraction result group 71T is data in which the same-day captured image 22T, that is, a set of the class, the position, and an image (hereinafter, referred to as a same-day BB image) 95T of the bounding box 90 of the object extracted from the same-day extraction result 70T is registered for each image ID of the same-day captured image 22T. Similarly, as shown in Fig. 10 as an example, the next-day extraction result group 71N is data in which the next-day extraction result 70N, that is, the class, the position, and an image (hereinafter, referred to as a next-day BB image) 95N of the bounding box 90 of the object extracted from the next-day captured image 22N ARE registered for each image ID of the next-day captured image 22N.

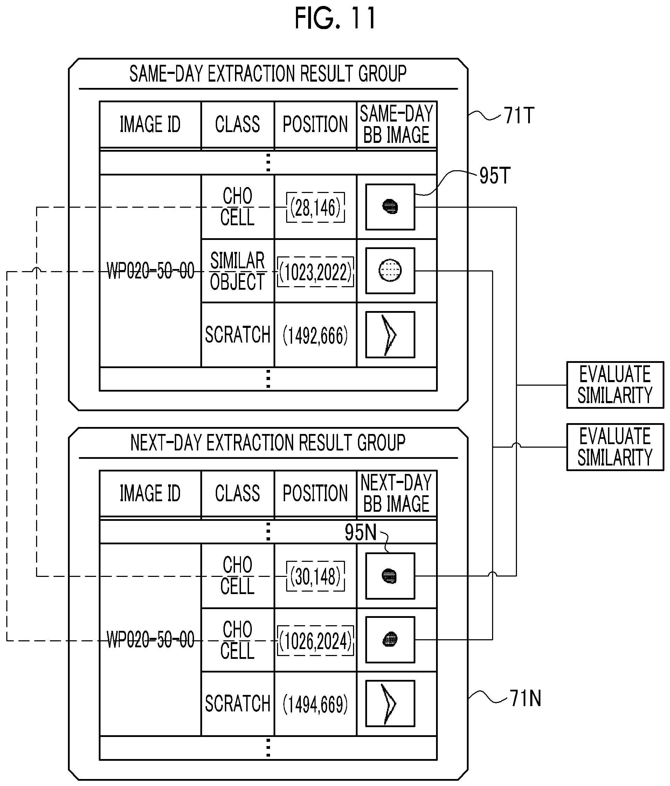

As shown in Fig. 11 as an example, the evaluation unit 63 searches for a second cell-like object extracted from the next-day captured image 22N, which corresponds in position to the first cell-like object extracted from the same-day captured image 22T, in the same-day captured image 22T and the next-day captured image 22N in which the same well 12 is imaged. The evaluation unit 63 evaluates similarity between the first cell-like object and the searched second cell-like object. Here, the second cell-like object "corresponding in position" to the first cell-like object includes not only the second cell-like object that is located in exactly the same position as the first cell-like object, but also the second cell-like object that is located within a preset range based on the position of the first cell-like object. The range is set in consideration of shift of the origin of the imaging position due to a mechanical error of the imaging device 20. The range has a margin of, for example, 10 CHO cells 13 in both X and Y directions based on the position of the first cell-like object.

Fig. 11 illustrates a case of evaluating the similarity between the first cell-like object with the class of "CHO cell" and the position of "(28, 146)" and the second cell-like object with the class of "CHO cell" and the position of "(30, 148)", which corresponds in position to the first cell-like object. In addition, Fig. 11 illustrates a case of evaluating the similarity between the first cell-like object with the class of "similar object" and the position of "(1023, 2022)" and the second cell-like object with the class of "CHO cell" and the position of "(1026, 2024)", which corresponds in position to the first cell-like object.

As shown in Fig. 12 as an example, the evaluation unit 63 includes a feature amount derivation unit 100 and a degree-of-similarity calculation unit 101. The same-day BB image 95T of the first cell-like object and the next-day BB image 95N of the second cell-like object corresponding in position to the first cell-like object are input to the feature amount derivation unit 100. The feature amount derivation unit 100 derives a same-day feature amount group 102T from the same-day BB image 95T and derives a next-day feature amount group 102N from the next-day BB image 95N.

The same-day feature amount group 102T includes a numerical value related to a pixel value, a numerical value related to the position, and a numerical value related to the shape of the first cell-like object in the same-day BB image 95T. Similarly, the next-day feature amount group 102N includes a numerical value related to a pixel value, a numerical value related to the position, and a numerical value related to the shape of the second cell-like object in the next-day BB image 95N. The numerical value related to the pixel value is, for example, an average value, a maximum value, a minimum value, a variance, and a standard deviation of the pixel values of the first cell-like object or the second cell-like object, and a difference between the pixel values of the first cell-like object or the second cell-like object and its surroundings. The numerical value related to the position is, for example, a distance of the first cell-like object or the second cell-like object from an edge of the well 12. The numerical value related to the shape is, for example, the size, circularity, or contour irregularity of the first cell-like object or the second cell-like object.

As described above, each of the same-day feature amount group 102T and next-day feature amount group 102N is a set of a plurality of types of feature amounts. The same-day feature amount group 102T and the next-day feature amount group 102N each having a plurality of types of feature amounts are referred to as a feature amount vector having the plurality of types of feature amounts as elements. In addition, a feature amount obtained by inputting the same-day BB image 95T and the next-day BB image 95N to an encoder unit of a machine learning model, such as an autoencoder, may be added to the same-day feature amount group 102T and the next-day feature amount group 102N.

The degree-of-similarity calculation unit 101 calculates a degree of similarity 103 between the first cell-like object of the same-day BB image 95T and the second cell-like object of the next-day BB image 95N based on the same-day feature amount group 102T and the next-day feature amount group 102N. The degree of similarity 103 is none other than the evaluation result 72.

More specifically, in a case in which the feature amount constituting the same-day feature amount group 102T is denoted by ZTi (i = 1, 2, 3, ..., N, where N is the total number of feature amounts), the feature amount constituting the next-day feature amount group 102N is denoted by ZNi, and the degree of similarity is denoted by S, the degree-of-similarity calculation unit 101 calculates the degree of similarity S according to Equation (1).

$$S = \Sigma\{(ZTi - ZNi)^2\}^{1/2} \dots (1)$$

A right side of Equation (1) is a square root of a sum of squares of a difference (ZTi - ZNi) between the feature amount ZTi and the feature amount ZNi, that is, a distance between a feature amount vector having the feature amount ZTi as an element and a feature amount vector having the feature amount ZNi as an element. Therefore, in a case in which the similarity between the first cell-like object and the second cell-like object is high, the distance between the feature amount vector having the feature amount ZTi as an element and the feature amount vector having the feature amount ZNi as an element is short, so that the value of the degree of similarity S is small. In other words, it can be said that the smaller the value of the degree of similarity S, the higher the similarity between the first cell-like object and the second cell-like object.

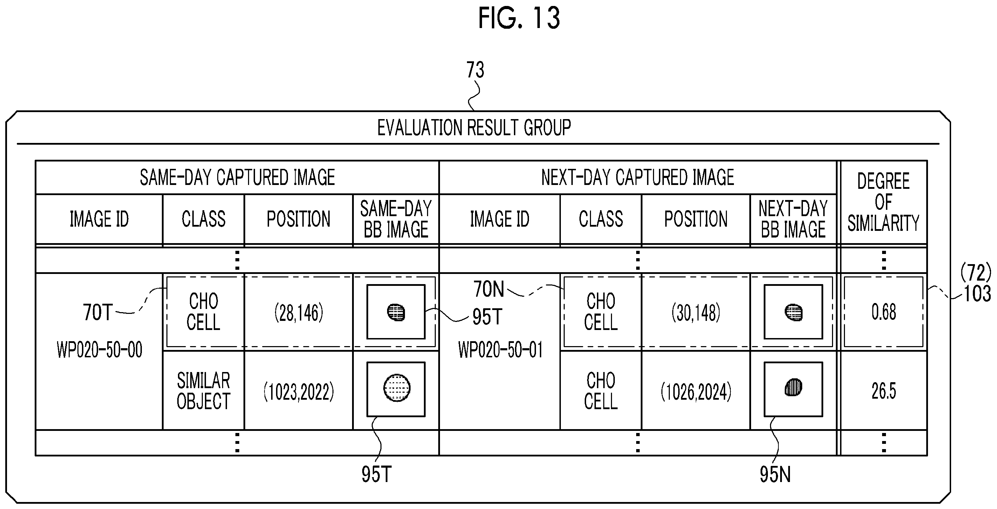

As shown in Fig. 13 as an example, the evaluation result group 73 is data in which the same-day extraction result 70T of the first cell-like object and the next-day extraction result 70N of the second cell-like object, for which the similarity has been evaluated, and the degree of similarity 103, which is the evaluation result 72, are registered for each image ID of the same-day captured image 22T and each image ID of the next-day captured image 22N.

The display control unit 64 displays the evaluation result display screen 110 shown in Fig. 14 as an example on the display 14. The evaluation result display screen 110 has a basic information display region 111 and an evaluation result display region 112. In the basic information display region 111, basic information such as a name of a development test of antibody pharmaceuticals, a type of a cell, and a seeding date of the cell is displayed. In the evaluation result display region 112, a list of objects shown in the same-day captured image 22T is displayed. More specifically, the evaluation result display region 112 has a plurality of divided regions 113 prepared for each same-day captured image 22T. In the divided region 113, the same-day BB images 95T of the first cell-like objects extracted from the same-day captured image 22T are displayed in a horizontal row. The evaluation result display region 112 is provided with a vertical scroll bar 114.

In the divided region 113, the same-day BB images 95T are arranged from left to right in descending order of the degree of similarity 103 to the second cell-like object, that is, in ascending order of the similarity to the second cell-like object. Numbers 115 are assigned to the same-day BB images 95T in ascending order of the similarity to the second cell-like object. In this way, by arranging the same-day BB images 95T from left to right in ascending order of the similarity to the second cell-like object and assigning the numbers 115 in ascending order of the similarity to the second cell-like object, the display control unit 64 displays the first cell-like object having relatively low similarity to the second cell-like object in an identifiable manner. The divided region 113 where the same-day BB images 95T do not fit is provided with a horizontal scroll bar 116.

In a case in which any same-day BB image 95T is selected by the user 16 on the evaluation result display screen 110, the display control unit 64 displays the comparison display screen 120 shown in Fig. 15 as an example on the display 14. The comparison display screen 120 has a basic information display region 121 similar to that of the evaluation result display screen 110, and a comparison display region 122. The comparison display region 122 is divided into a same-day image display region 123 on a left side and a next-day image display region 124 on a right side. In the same-day image display region 123, the same-day BB image 95T selected by the user 16 on the evaluation result display screen 110 and the same-day captured image 22T from which the same-day BB image 95T is derived are displayed. In the next-day image display region 124, the next-day BB image 95N of the second cell-like object corresponding in position to the first cell-like object of the same-day BB image 95T selected by the user 16 on the evaluation result display screen 110, and the next-day captured image 22N from which the next-day BB image 95N is derived are displayed. The distinguished class is displayed in the same-day BB image 95T and the next-day BB image 95N. As described above, the display control unit 64 displays the first cell-like object and the second cell-like object corresponding in position to the first cell-like object side by side.

In the same-day image display region 123, the bounding box 90 of the same-day BB image 95T is displayed in the same-day captured image 22T. Then, a leader line 125, which is a broken line, is drawn from the bounding box 90 to the same-day BB image 95T. The bounding box 90 and the leader line 125 indicate the position of the first cell-like object in the same-day captured image 22T. That is, the bounding box 90 and the leader line 125 are examples of a "first indicator" according to the technology of the present disclosure.

Similarly, in the next-day image display region 124, the bounding box 90 of the next-day BB image 95N is displayed in the next-day captured image 22N. Then, a leader line 126, which is a broken line, is drawn from the bounding box 90 to the next-day BB image 95N. The bounding box 90 and the leader line 126 indicate the position of the second cell-like object in the next-day captured image 22N. That is, the bounding box 90 and the leader line 126 are examples of a "second indicator" according to the technology of the present disclosure. The first indicator and the second indicator are not limited to the above examples, and may be an arrow or the like.

Determination buttons 127A and 127B are provided below the comparison display region 122. The user 16 observes the same-day BB image 95T and the next-day BB image 95N in the comparison display region 122. In a case in which the object shown in the same-day BB image 95T and the next-day BB image 95N is determined to be the CHO cell 13, the determination button 127A is selected, and, in a case in which the object is determined not to be the CHO cell 13, the determination button 127B is selected. The results of the determination made by the determination buttons 127A and 127B as to whether or not the object shown in the same-day BB image 95T and the next-day BB image 95N is the CHO cell 13 are stored in association with the same-day captured image 22T and the next-day captured image 22N.

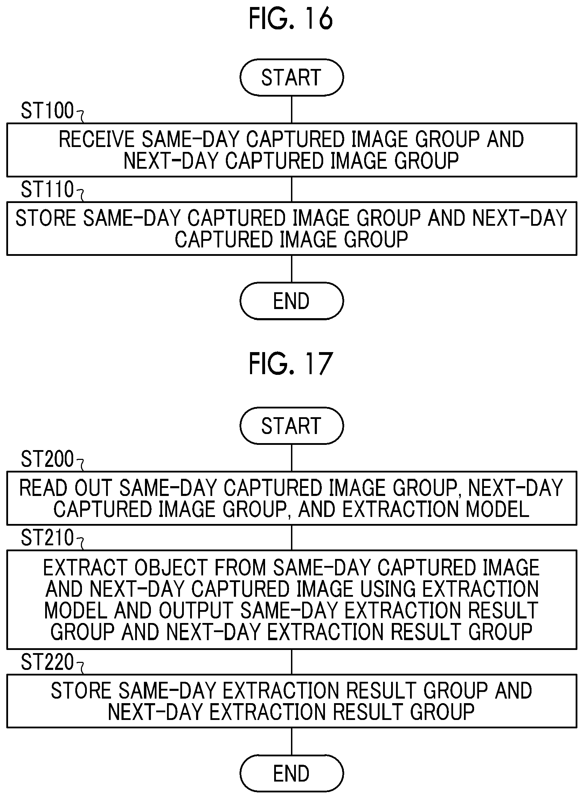

Next, an operation of the configuration described above will be described with reference to flowcharts shown in Figs. 16 to 18 as an example. In a case in which the operation program 50 is activated in the determination support device 10, as shown in Fig. 5, the CPU 42 of the determination support device 10 functions as the receiving unit 60, the RW control unit 61, the extraction unit 62, the evaluation unit 63, and the display control unit 64.

First, the receiving unit 60 receives the same-day captured image group 23T and the next-day captured image group 23N from the imaging device 20 (step ST100 in Fig. 16). The same-day captured image group 23T and the next-day captured image group 23N are output from the receiving unit 60 to the RW control unit 61 and are stored in the storage 40 under the control of the RW control unit 61 (step ST110).

The same-day captured image group 23T, the next-day captured image group 23N, and the extraction model 51 are read out from the storage 40 by the RW control unit 61 (step ST200 in Fig. 17). The same-day captured image group 23T, the next-day captured image group 23N, and the extraction model 51 are output from the RW control unit 61 to the extraction unit 62. In addition, the same-day captured image group 23T and the next-day captured image group 23N are output from the RW control unit 61 to the display control unit 64.

In the extraction unit 62, as shown in Figs. 6 to 8, various objects are extracted from the same-day captured image 22T and the next-day captured image 22N by the extraction model 51, and the same-day extraction result 70T and the next-day extraction result 70N are output from the extraction model 51. Then, the same-day extraction result group 71T, which is a set of the same-day extraction results 70T, and the next-day extraction result group 71N, which is a set of the next-day extraction results 70N, are output from the extraction unit 62 to the RW control unit 61 (step ST210). The same-day extraction result group 71T and the next-day extraction result group 71N are stored in the storage 40 under the control of the RW control unit 61 (step ST220).

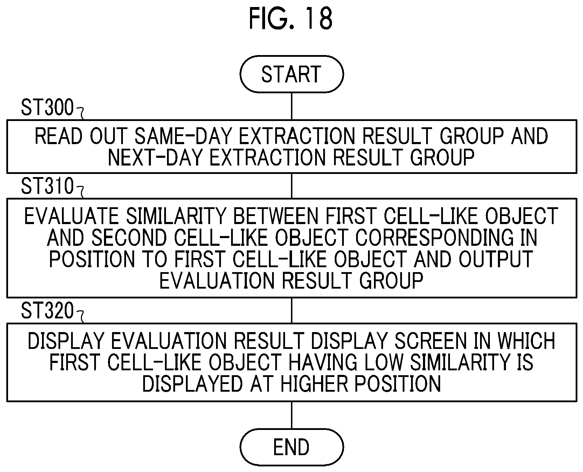

The same-day extraction result group 71T and the next-day extraction result group 71N are read out from the storage 40 by the RW control unit 61 (step ST300 in Fig. 18). The same-day extraction result group 71T and the next-day extraction result group 71N are output from the RW control unit 61 to the evaluation unit 63.

As shown in Fig. 11, the evaluation unit 63 searches for the second cell-like object extracted from the next-day captured image 22N, which corresponds in position to the first cell-like object extracted from the same-day captured image 22T. Then, the similarity between the first cell-like object and the searched second cell-like object is evaluated (step ST310). More specifically, as shown in Fig. 12, the same-day feature amount group 102T of the same-day BB image 95T and the next-day feature amount group 102N of the next-day BB image 95N are derived by the feature amount derivation unit 100. Next, the degree of similarity 103, which is the distance between the feature amount vector represented by the same-day feature amount group 102T and the feature amount vector represented by the next-day feature amount group 102N, is calculated by the degree-of-similarity calculation unit 101. Then, the evaluation result group 73 including the degree of similarity 103 as the evaluation result 72 is output from the evaluation unit 63 to the display control unit 64 (step ST310).

As shown in Fig. 14, under the control of the display control unit 64, the evaluation result display screen 110 in which the same-day BB images 95T of the first cell-like objects having relatively low similarity are displayed at a higher position is displayed on the display 14 (step ST320).

In a case in which the user 16 selects the same-day BB image 95T on the evaluation result display screen 110, the comparison display screen 120 shown in Fig. 15 is displayed on the display 14 under the control of the display control unit 64. As a result, the same-day BB image 95T and the next-day BB image 95N are provided for viewing by the user 16.

As described above, the CPU 42 of the determination support device 10 comprises the receiving unit 60, the extraction unit 62, the evaluation unit 63, and the display control unit 64. The receiving unit 60 acquires the same-day captured image 22T, which is the captured image 22 of the well 12 on the day the CHO cell 13 is seeded, and the next-day captured image 22N, which is the captured image 22 of the well 12 on the day after the CHO cell 13 is seeded, by receiving the captured images from the imaging device 20. The extraction unit 62 extracts the cell-like object, which is any of the CHO cell 13 or the similar object 93 that is morphologically similar to the CHO cell 13, from each of the same-day captured image 22T and the next-day captured image 22N.

The evaluation unit 63 evaluates the similarity between the first cell-like object, which is the cell-like object extracted from the same-day captured image 22T, and the second cell-like object, which is the cell-like object extracted from the next-day captured image 22N and corresponds in position to the first cell-like object. The display control unit 64 displays the first cell-like object having relatively low similarity in an identifiable manner.

The floating CHO cell 13F is shown in a manner that is far from the manner in which the typical CHO cell 13 is shown after being sedimented on the bottom surface of the well 12. Therefore, the probability that the first cell-like object, which has relatively low similarity to the second cell-like object including the CHO cell 13 that will certainly have sedimented on the bottom surface of the well 12 on the day after seeding, is the floating CHO cell 13F is high. The floating CHO cell 13F causes an error in the determination of the cellular monoclonality of the CHO cell 13. Therefore, in a case in which the first cell-like object having relatively low similarity to the second cell-like object is displayed in an identifiable manner, the first cell-like object to be checked intensively can be easily understood at a glance, so that the user 16 can make the determination more efficiently. From the above, the technology of the present disclosure can reduce the time and effort for determining the cellular monoclonality. In addition, it is also possible to reduce the risk of determining that no CHO cell 13 is present even though the floating CHO cell 13F is actually present.

As shown in Fig. 15, the display control unit 64 displays the first cell-like object and the second cell-like object corresponding in position to the first cell-like object side by side. Therefore, the user 16 can compare the first cell-like object and the second cell-like object corresponding in position to the first cell-like object in detail and determine whether or not the first cell-like object and the second cell-like object are the CHO cells 13.

As shown in Fig. 15, the display control unit 64 displays the bounding box 90 and the leader line 125 as the first indicator indicating the position of the first cell-like object in the same-day captured image 22T. In addition, the display control unit 64 displays the bounding box 90 and the leader line 126 as the second indicator indicating the position of the second cell-like object in the next-day captured image 22N. Therefore, the user 16 can easily check the positions of the first cell-like object and the second cell-like object in the same-day captured image 22T and the next-day captured image 22N. The user 16 can easily check whether or not the first cell-like object and the second cell-like object are truly present at corresponding positions.

The cell is the CHO cell 13 into which the antibody gene 19 is incorporated. The CHO cell 13 has a proliferation cycle of approximately one day. Therefore, this is suitable for the technology of the present disclosure, which evaluates the similarity between the first cell-like object of the same-day captured image 22T and the second cell-like object of the next-day captured image 22N.

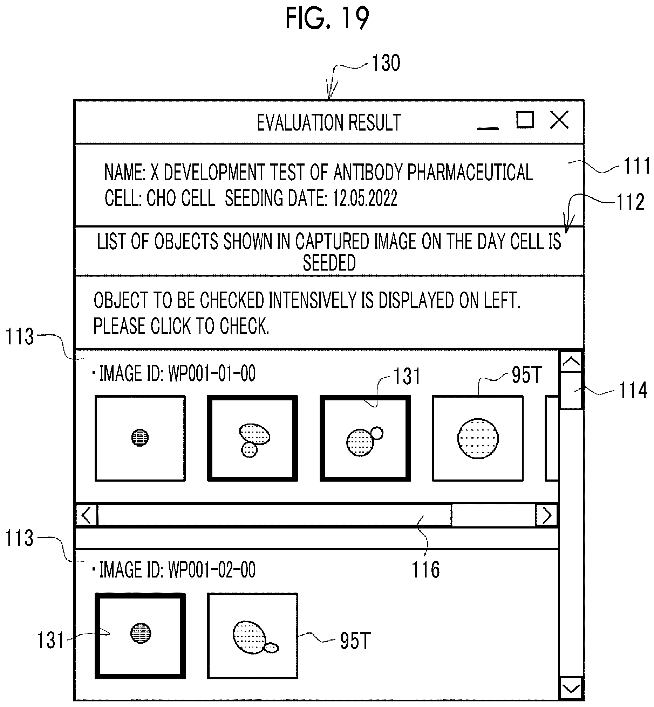

The method of displaying the first cell-like object having relatively low similarity to the second cell-like object in an identifiable manner is not limited to a form in which the same-day BB images 95T are arranged from left to right in ascending order of the similarity to the second cell-like object and the numbers 115 are assigned in ascending order of the similarity to the second cell-like object as in the evaluation result display screen 110 shown in Fig. 14. As shown in an evaluation result display screen 130 shown in Fig. 19 as an example, a highlight display frame 131 may be displayed on the same-day BB image 95T of the first cell-like object whose similarity to the second cell-like object is lower than a preset threshold value (the degree of similarity 103 is higher than the preset threshold value) to display the first cell-like object having relatively low similarity to the second cell-like object in an identifiable manner.

As the method of displaying the first cell-like object having relatively low similarity to the second cell-like object in an identifiable manner, for example, a form in which only the same-day BB image 95T of the first cell-like object whose similarity to the second cell-like object is lower than a threshold value is displayed, or a form in which only the same-day BB image 95T of the first cell-like object whose similarity to the second cell-like object is in a rank equal to or lower than a preset rank (a descending rank of the degree of similarity 103 is equal to or higher than a preset rank) is displayed may be employed. In addition, a form may be employed in which the same-day BB image 95T of the first cell-like object whose similarity to the second cell-like object is lower than the threshold value is displayed to be larger than the same-day BB image 95T of the other first cell-like object.

[Second Embodiment]

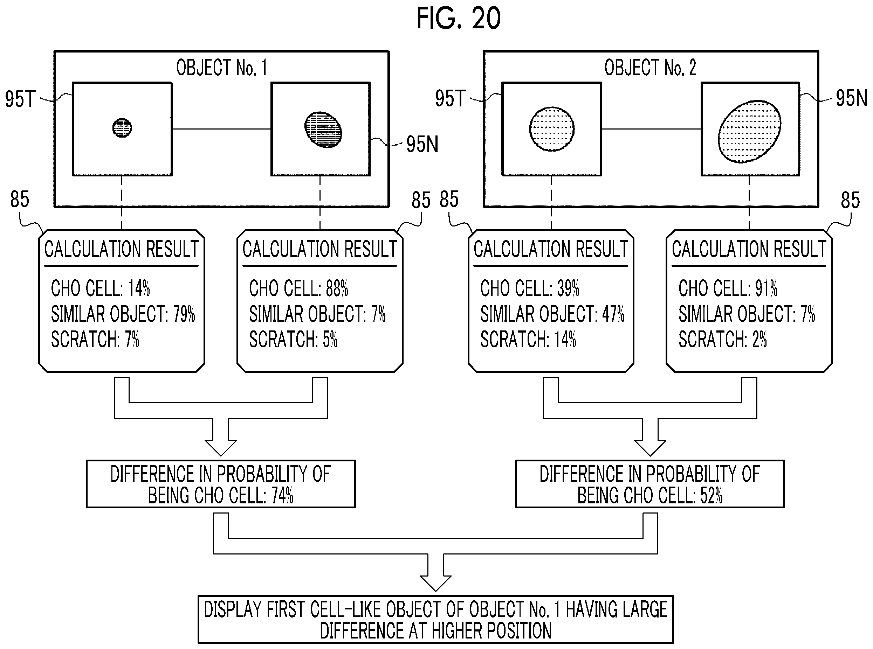

Fig. 20 shows an example in which a display form of the first cell-like object is changed according to a difference between the probability that the first cell-like object is the CHO cell 13 and the probability that the second cell-like object corresponding in position to the first cell-like object is the CHO cell 13. In this case, the display control unit 64 displays the same-day BB image 95T of the first cell-like object having a relatively large difference in the probability of being the CHO cell 13 at a higher position on the evaluation result display screen. Fig. 20 illustrates a case in which a difference in the probability that an object No. 1 is the CHO cell 13 is 74% and a difference in the probability that an object No. 2 is the CHO cell 13 is 52%. In this case, the display control unit 64 displays the same-day BB image 95T of the first cell-like object of the object No. 1 at a higher position than the same-day BB image 95T of the first cell-like object of the object No. 2.

In a case in which the difference between the probability that the first cell-like object is the CHO cell 13 and the probability that the second cell-like object corresponding in position to the first cell-like object is the CHO cell 13 is large, the probability that the first cell-like object is the floating CHO cell 13F is high. Therefore, in a case in which the same-day BB image 95T of the first cell-like object having a relatively large difference in the probability of being the CHO cell 13 is displayed at a higher position, the first cell-like object to be checked intensively is seen first, so that the user 16 can make the determination more efficiently.

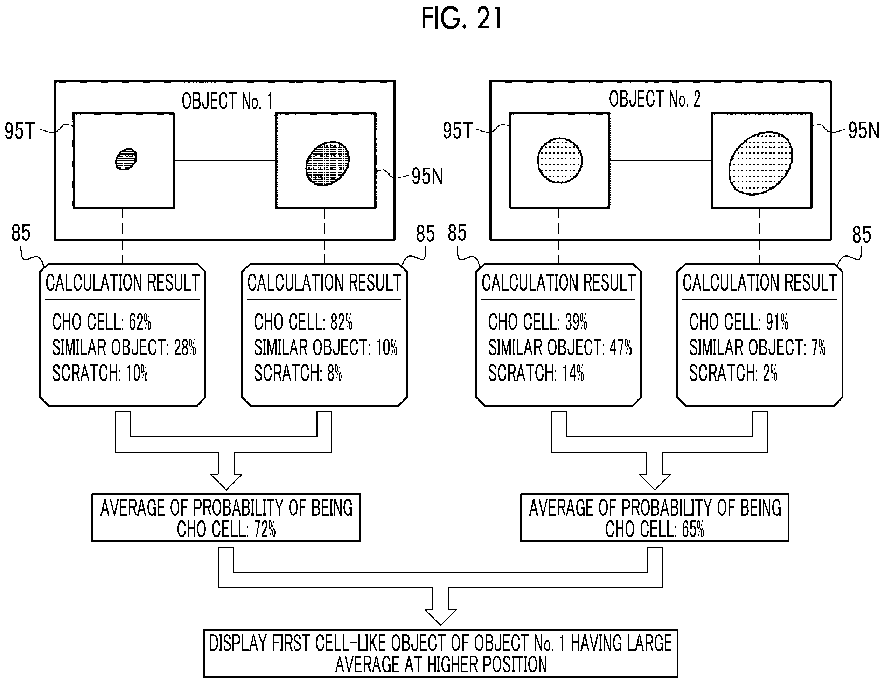

Fig. 21 shows an example in which a display form of the first cell-like object is changed according to an average between the probability that the first cell-like object is the CHO cell 13 and the probability that the second cell-like object corresponding in position to the first cell-like object is the CHO cell 13. In this case, the display control unit 64 displays, for example, the same-day BB image 95T of the first cell-like object having a relatively large average of the probability of being the CHO cell 13 at a higher position on the evaluation result display screen. Fig. 21 illustrates a case in which an average of the probability that an object No. 1 is the CHO cell 13 is 72% and an average of the probability that an object No. 2 is the CHO cell 13 is 65%. In this case, the display control unit 64 displays the same-day BB image 95T of the first cell-like object of the object No. 1 at a higher position than the same-day BB image 95T of the first cell-like object of the object No. 2.

In a case in which the average of the probability that the first cell-like object is the CHO cell 13 and the probability that the second cell-like object corresponding in position to the first cell-like object is the CHO cell 13 is large, the probability that the first cell-like object and the second cell-like object are not the debris 91 but the CHO cell 13 is high. Therefore, in a case where the same-day BB image 95T of the first cell-like object having a relatively large average of the probabilities of the CHO cells 13 is displayed at a higher position, the first cell-like object to be confirmed intensively is seen first, so that the user 16 can make the determination more efficiently.

The display form according to the probability of being the CHO cell 13 in Figs. 20 and 21 is applied to the first cell-like object whose similarity to the second cell-like object is equal to or higher than a preset threshold value (the degree of similarity 103 is equal to or lower than the preset threshold value) and whose similarity to the second cell-like object is relatively high. For the first cell-like object whose similarity to the second cell-like object is less than the threshold value and whose similarity to the second cell-like object is relatively low, the display form according to the similarity shown in the first embodiment is applied.

As described above, in the second embodiment, the display form of the first cell-like object is changed according to the probability of being the CHO cell 13 which is calculated by the calculation unit 82 of the extraction model 51. Therefore, it is possible to further reduce the time and effort for determining the cellular monoclonality.

On the evaluation result display screen, not only the same-day BB image 95T but also the next-day BB image 95N may be displayed in a list. The next-day BB image 95N may be displayed in an identifiable manner by, for example, arranging the BB images in order of relatively low similarity to the first cell-like object.

An extraction model used for the same-day captured image 22T and an extraction model used for the next-day captured image 22N may be prepared separately. The extraction model used for the same-day captured image 22T is a model that extracts the CHO cell 13 and the similar object 93 from the same-day captured image 22T, as with the extraction model 51 of the first embodiment. On the other hand, the extraction model used for the next-day captured image 22N may be a model that extracts only the CHO cell 13.

In each of the above-described embodiments, the similar object 93 includes the debris 91, but the present disclosure is not limited thereto. The debris 91 may be excluded from the similar object 93 and may be distinguished as a class independent of the similar object 93, such as the scratch 92.

The hardware configuration of the computer constituting the determination support device 10 according to the technology of the present disclosure can be modified in various ways. For example, the determination support device 10 may be configured of a plurality of computers that are separated as hardware for the purpose of improving processing capability and reliability. For example, the functions of the receiving unit 60 and the extraction unit 62 and the functions of the evaluation unit 63 and the display control unit 64 are assigned to two computers in a distributed manner. In this case, the determination support device 10 is configured of the two computers.

As described above, the hardware configuration of the computer of the determination support device 10 can be changed as appropriate depending on required performance such as processing capacity, safety, and reliability. Further, it goes without saying that, in addition to the hardware, an application program such as the operation program 50 can be duplicated or distributed and stored in a plurality of storages for the purpose of securing the safety and the reliability.

In each of the above-described embodiments, for example, the following various processors can be used as a hardware structure of processing units that execute various types of processing, such as the receiving unit 60, the RW control unit 61, the extraction unit 62, the evaluation unit 63, the display control unit 64, the feature amount derivation unit 100, and the degree-of-similarity calculation unit 101. As described above, the various processors include, in addition to the CPU 42 that is a general-purpose processor which executes software (operation program 50) to function as various processing units, a programmable logic device (PLD) that is a processor of which a circuit configuration can be changed after manufacture, such as a field programmable gate array (FPGA), and a dedicated electrical circuit that is a processor having a circuit configuration which is designed for exclusive use to execute specific processing, such as an application specific integrated circuit (ASIC).

One processing unit may be configured of one of the various processors or may be configured of a combination of two or more processors of the same type or different types (for example, a combination of a plurality of FPGAs and/or a combination of a CPU and an FPGA). In addition, a plurality of processing units may be configured of one processor.

As an example of configuring the plurality of processing units with one processor, first, there is a form in which, as typified by computers such as a client and a server, one processor is configured of a combination of one or more CPUs and software and the processor functions as the plurality of processing units. Second, there is a form in which, as typified by a system on chip (SoC) and the like, a processor that implements functions of an entire system including the plurality of processing units with one integrated circuit (IC) chip is used. As described above, various processing units are configured by using one or more of the various processors as a hardware structure.

In addition, more specifically, an electric circuit (circuitry) in which circuit elements, such as semiconductor elements, are combined can be used as the hardware structure of these various processors.

It is possible to understand the technologies described in following Appendices from the above description.

[Appendix 1]

A determination support device that supports determination of monoclonality of a cell seeded in a container, the determination support device comprising:

a processor,
in which the processor is configured to

acquire a same-day captured image, which is a captured image of the container on the day the cell is seeded, and a next-day captured image, which is a captured image of the container on the day after the cell is seeded,
extract a cell-like object, which is any of the cell or a similar object that is morphologically similar to the cell, from each of the same-day captured image and the next-day captured image,
evaluate similarity between a first cell-like object, which is the cell-like object extracted from the same-day captured image, and a second cell-like object, which is the cell-like object extracted from the next-day captured image and corresponds in position to the first cell-like object, and

display the first cell-like object of which the similarity is relatively low in an identifiable manner.

[Appendix 2]

The determination support device according to Appendix 1,
in which the processor is configured to display the first cell-like object and the second cell-like object that corresponds in position to the first cell-like object side by side.

[Appendix 3]

The determination support device according to Appendix 2,
in which the processor is configured to

display a first indicator indicating a position of the first cell-like object in the same-day captured image, and display a second indicator indicating a position of the second cell-like object in the next-day captured image.

[Appendix 4]

The determination support device according to any one of Appendices 1 to 3,
in which the processor is configured to

extract the cell-like object using a machine learning model that distinguishes between a plurality of classes including the cell and the similar object, and change a display form of the first cell-like object according to a probability of the cell calculated by the machine learning model.

[Appendix 5]

The determination support device according to any one of Appendices 1 to 4,
in which the cell is a Chinese hamster ovary cell into which an antibody gene is incorporated.

In the technology of the present disclosure, the above-described various embodiments and/or various modification examples may be combined with each other as appropriate. In addition, the present disclosure is not limited to the above-described embodiments, and various configurations can be adopted without departing from the gist of the present disclosure. Further, the technology of the present disclosure extends to a storage medium that non-transitorily stores a program in addition to the program.

The above descriptions and illustrations are detailed descriptions of portions related to the technology of the present disclosure and are merely examples of the technology of the present disclosure. For example, description related to the above configurations, functions, actions, and effects is description related to an example of configurations, functions, actions, and effects of the parts according to the technology of the present disclosure. Thus, it is needless to say that unnecessary portions may be deleted, new elements may be added, or replacement may be made to the content of the above description and the content of the drawings without departing from the gist of the technique of the present disclosure. Further, in order to avoid complications and facilitate understanding of the parts related to the technology of the present disclosure, descriptions of common general knowledge and the like that do not require special descriptions for enabling the implementation of the technology of the present disclosure are omitted, in the contents described and shown above.

In the present specification, the term "A and/or B" is synonymous with the term "at least one of A or B". That is, the term "A and/or B" means only A, only B, or a combination of A and B. In addition, in the present specification, the same approach as "A and/or B" is applied to a case in which three or more matters are represented by connecting the matters with "and/or".

All documents, patent applications, and technical standards mentioned in the present specification are incorporated herein by reference to the same extent as in a case in which each document, each patent application, and each technical standard are specifically and individually described by being incorporated by reference.

**Claims**

1.  A determination support device that supports determination of monoclonality of a cell seeded in a container, the determination support device comprising:

    a processor,
    wherein the processor is configured to

        acquire a same-day captured image, which is a captured image of the container on the day the cell is seeded, and a next-day captured image, which is a captured image of the container on the day after the cell is seeded,
        extract a cell-like object, which is any of the cell or a similar object that is morphologically similar to the cell, from each of the same-day captured image and the next-day captured image,
        evaluate similarity between a first cell-like object, which is the cell-like object extracted from the same-day captured image, and a second cell-like object, which is the cell-like object extracted from the next-day captured image and corresponds in position to the first cell-like object, and
        display the first cell-like object of which the similarity is relatively low in an identifiable manner.

2. The determination support device according to claim 1,
   wherein the processor is configured to display the first cell-like object and the second cell-like object that corresponds in position to the first cell-like object side by side.

3. The determination support device according to claim 2,
   wherein the processor is configured to

   display a first indicator indicating a position of the first cell-like object in the same-day captured image, and
   display a second indicator indicating a position of the second cell-like object in the next-day captured image.

4. The determination support device according to claim 1,
   wherein the processor is configured to

   extract the cell-like object using a machine learning model that distinguishes between a plurality of classes including the cell and the similar object, and
   change a display form of the first cell-like object according to a probability of the cell calculated by the machine learning model.

5. The determination support device according to claim 1,
   wherein the cell is a Chinese hamster ovary cell into which an antibody gene is incorporated.

6. An operation method of a determination support device that supports determination of monoclonality of a cell seeded in a container, the operation method comprising:

   acquiring a same-day captured image, which is a captured image of the container on the day the cell is seeded, and a next-day captured image, which is a captured image of the container on the day after the cell is seeded;
   extracting a cell-like object, which is any of the cell or a similar object that is morphologically similar to the cell, from each of the same-day captured image and the next-day captured image;
   evaluating similarity between a first cell-like object, which is the cell-like object extracted from the same-day captured image, and a second cell-like object, which is the cell-like object extracted from the next-day captured image and corresponds in position to the first cell-like object; and
displaying the first cell-like object of which the similarity is relatively low in an identifiable manner.

7. An operation program of a determination support device that supports determination of monoclonality of a cell seeded in a container, the operation program causing a computer to execute a process comprising:

   acquiring a same-day captured image, which is a captured image of the container on the day the cell is seeded, and a next-day captured image, which is a captured image of the container on the day after the cell is seeded;
   extracting a cell-like object, which is any of the cell or a similar object that is morphologically similar to the cell, from each of the same-day captured image and the next-day captured image;
   evaluating similarity between a first cell-like object, which is the cell-like object extracted from the same-day captured image, and a second cell-like object, which is the cell-like object extracted from the next-day captured image and corresponds in position to the first cell-like object; and
   displaying the first cell-like object of which the similarity is relatively low in an identifiable manner.

FIG. 1

## FIG. 2

ON THE DAY CELL
IS SEEDED

23T

SAME-DAY
CAPTURED IMAGE GROUP

⋮ 22T

SAME-DAY
CAPTURED IMAGE

IMAGE ID: WP010-01-00

⋮

10

20

## FIG. 3

ON THE DAY AFTER
CELL IS SEEDED

23N

NEXT-DAY
CAPTURED IMAGE GROUP

⋮ 22N

NEXT-DAY
CAPTURED IMAGE

IMAGE ID: WP010-01-01

⋮

10

20

# FIG. 4

# FIG. 5

IMAGING DEVICE ～20

CAPTURED IMAGE GROUP ～23

10

40

CPU

RECEIVING UNIT ～60

CAPTURED IMAGE GROUP 23

STORAGE

CAPTURED IMAGE GROUP

23

EXTRACTION UNIT 62

SAME-DAY CAPTURED IMAGE GROUP ～23T

NEXT-DAY CAPTURED IMAGE GROUP ～23N

①

EXTRACTION MODEL 51

RW CONTROL UNIT

EXTRACTION MODEL 51

SAME-DAY EXTRACTION RESULT GROUP ～71T

NEXT-DAY EXTRACTION RESULT GROUP ～71N

SAME-DAY EXTRACTION RESULT GROUP ～71T

NEXT-DAY EXTRACTION RESULT GROUP ～71N

63 EVALUATION UNIT

SAME-DAY EXTRACTION RESULT GROUP ～71T

NEXT-DAY EXTRACTION RESULT GROUP ～71N

EVALUATION RESULT GROUP 73

61

DISPLAY CONTROL UNIT 64 ①

50

DISPLAY ～14

42

OPERATION PROGRAM

DETERMINATION SUPPORT DEVICE

## FIG. 6

22(22T,22N) —~ CAPTURED
IMAGE

51

EXTRACTION
MODEL

80 —~ ENCODER
UNIT

FEATURE
AMOUNT —~ 84

81 —~ DECODER
UNIT

82 —~ CALCULATION
UNIT

CALCULATION RESULT

85

CHO CELL: 27%
SIMILAR OBJECT: 68%
SCRATCH: 5%

83 —~ OUTPUT UNIT

EXTRACTION RESULT

70(70T,70N) —~

SIMILAR OBJECT

# FIG. 7

SAME-DAY CAPTURED IMAGE

92

13

(93)91

13F(93)

22T

EXTRACTION MODEL — 51

SAME-DAY EXTRACTION RESULT

SCRATCH

90

CHO CELL

90

DEBRIS

SIMILAR OBJECT

70T

CHO CELL: (450,382)
SIMILAR OBJECT 1: (472,967)
SIMILAR OBJECT 2: (882,732)
SCRATCH: (911,264)

# FIG. 8

NEXT-DAY CAPTURED IMAGE

92

13

(93)91

13

22N

EXTRACTION MODEL — 51

NEXT-DAY EXTRACTION RESULT

90 → SCRATCH

CHO CELL

90

SIMILAR OBJECT

CHO CELL

70N

CHO CELL 1: (447,380)
CHO CELL 2: (470,961)
SIMILAR OBJECT: (879,730)
SCRATCH: (909,260)

# FIG. 9

| | | | SAME-DAY EXTRACTION RESULT GROUP | | |
|---|---|---|---|---|---|
| IMAGE ID | CLASS | POSITION | SAME-DAY BB IMAGE | | |
| ⋮ | | | | | |
| WP020-50-00 | CHO CELL | (28,146) | | 70T<br>13 |
| | SIMILAR OBJECT | (1023,2022) | | 71T<br>13F(93)<br>95T |
| | SCRATCH | (1492,666) | | 92 |
| ⋮ | | | | | |

# FIG. 10

| | | | NEXT-DAY EXTRACTION RESULT GROUP | | |
|---|---|---|---|---|---|
| IMAGE ID | CLASS | POSITION | NEXT-DAY BB IMAGE | | |
| ⋮ | | | | | |
| WP020-50-01 | CHO CELL | (30,148) | | 70N<br>13 |
| | CHO CELL | (1026,2024) | | 71N<br>13<br>95N |
| | SCRATCH | (1494,669) | | 92 |
| ⋮ | | | | | |

# FIG. 11

## SAME-DAY EXTRACTION RESULT GROUP ~71T

| IMAGE ID | CLASS | POSITION | SAME-DAY BB IMAGE |
|---|---|---|---|
| ⋮ | | | |
| WP020-50-00 | CHO CELL | (28,146) | ●  ~95T |
| | SIMILAR OBJECT | (1023,2022) | ◉ |
| | SCRATCH | (1492,666) | ❯ |
| ⋮ | | | |

## NEXT-DAY EXTRACTION RESULT GROUP

| IMAGE ID | CLASS | POSITION | NEXT-DAY BB IMAGE |
|---|---|---|---|
| ⋮ | | | 95N |
| WP020-50-00 | CHO CELL | (30,148) | ● |
| | CHO CELL | (1026,2024) | ● |
| | SCRATCH | (1494,669) | ❯ |
| ⋮ | | | |

~71N

EVALUATE SIMILARITY

EVALUATE SIMILARITY

# FIG. 12

EVALUATION UNIT

~63

SAME-DAY
BB IMAGE ~95T

NEXT-DAY
BB IMAGE

95N

100

FEATURE AMOUNT DERIVATION UNIT

102T    102N

SAME-DAY FEATURE
AMOUNT GROUP

NEXT-DAY FEATURE
AMOUNT GROUP

101

DEGREE-OF-SIMILARITY
CALCULATION UNIT

DEGREE OF
SIMILARITY ~103(72)

# FIG. 13

73

| EVALUATION RESULT GROUP | | | | | | | | |
|---|---|---|---|---|---|---|---|---|
| SAME-DAY CAPTURED IMAGE | | | | NEXT-DAY CAPTURED IMAGE | | | | DEGREE OF SIMILARITY |
| IMAGE ID | CLASS | POSITION | SAME-DAY BB IMAGE | IMAGE ID | CLASS | POSITION | NEXT-DAY BB IMAGE | |
| ⋮ | | ⋮ | | ⋮ | | ⋮ | | ⋮ |
| 70T<br>WP020-50-00 | CHO CELL | (28,146) |  | 70N<br>95T<br>WP020-50-01 | CHO CELL | (30,148) |  | 0.68 |
| | SIMILAR OBJECT | (1023,2022) |  | | CHO CELL | (1026,2024) |  | 26.5 |
| | | ⋮ | | | | ⋮ | | ⋮ |

95T          95N

(72)
103

# FIG. 14

EVALUATION RESULT  _ □ ✕

NAME: X DEVELOPMENT TEST OF ANTIBODY PHARMACEUTICAL
CELL: CHO CELL  SEEDING DATE: 12.05.2022

LIST OF OBJECTS SHOWN IN CAPTURED IMAGE ON THE DAY CELL
IS SEEDED

OBJECT TO BE CHECKED INTENSIVELY IS DISPLAYED ON LEFT.
PLEASE CLICK TO CHECK.

· IMAGE ID: WP001-01-00

95T

1    2    3    4

· IMAGE ID: WP001-02-00

116    115

95T

1    2

115

# FIG. 15

COMPARISON DISPLAY

NAME: X DEVELOPMENT TEST OF ANTIBODY PHARMACEUTICAL   CELL: CHO CELL
SEEDING DATE: 12.05.2022

WELL PLATE No. 01   WELL 01   OBJECT No. 3

90

90

SIMILAR OBJECT

CHO CELL

127A DETERMINED TO BE CELL

127B DETERMINED NOT TO BE CELL

EP 4 640 816 A1

# FIG. 16

START

ST100

RECEIVE SAME-DAY CAPTURED IMAGE GROUP AND NEXT-DAY CAPTURED IMAGE GROUP

ST110

STORE SAME-DAY CAPTURED IMAGE GROUP AND NEXT-DAY CAPTURED IMAGE GROUP

END

# FIG. 17

START

ST200

READ OUT SAME-DAY CAPTURED IMAGE GROUP, NEXT-DAY CAPTURED IMAGE GROUP, AND EXTRACTION MODEL

ST210

EXTRACT OBJECT FROM SAME-DAY CAPTURED IMAGE AND NEXT-DAY CAPTURED IMAGE USING EXTRACTION MODEL AND OUTPUT SAME-DAY EXTRACTION RESULT GROUP AND NEXT-DAY EXTRACTION RESULT GROUP

ST220

STORE SAME-DAY EXTRACTION RESULT GROUP AND NEXT-DAY EXTRACTION RESULT GROUP

END

# FIG. 18

START

ST300
READ OUT SAME-DAY EXTRACTION RESULT GROUP AND NEXT-DAY EXTRACTION RESULT GROUP

ST310
EVALUATE SIMILARITY BETWEEN FIRST CELL-LIKE OBJECT AND SECOND CELL-LIKE OBJECT CORRESPONDING IN POSITION TO FIRST CELL-LIKE OBJECT AND OUTPUT EVALUATION RESULT GROUP

ST320
DISPLAY EVALUATION RESULT DISPLAY SCREEN IN WHICH FIRST CELL-LIKE OBJECT HAVING LOW SIMILARITY IS DISPLAYED AT HIGHER POSITION

END

## FIG. 19

EVALUATION RESULT

NAME: X DEVELOPMENT TEST OF ANTIBODY PHARMACEUTICAL
CELL: CHO CELL  SEEDING DATE: 12.05.2022

LIST OF OBJECTS SHOWN IN CAPTURED IMAGE ON THE DAY CELL IS SEEDED

OBJECT TO BE CHECKED INTENSIVELY IS DISPLAYED ON LEFT.
PLEASE CLICK TO CHECK.

· IMAGE ID: WP001-01-00

· IMAGE ID: WP001-02-00

# FIG. 20

**OBJECT No. 1**

95T — [ ] — 95N

85 —

**CALCULATION RESULT**

CHO CELL: 14%
SIMILAR OBJECT: 79%
SCRATCH: 7%

— 85

**CALCULATION RESULT**

CHO CELL: 88%
SIMILAR OBJECT: 7%
SCRATCH: 5%

**DIFFERENCE IN PROBABILITY OF BEING CHO CELL: 74%**

**OBJECT No. 2**

95T — [ ] — 95N

85 —

**CALCULATION RESULT**

CHO CELL: 39%
SIMILAR OBJECT: 47%
SCRATCH: 14%

— 85

**CALCULATION RESULT**

CHO CELL: 91%
SIMILAR OBJECT: 7%
SCRATCH: 2%

**DIFFERENCE IN PROBABILITY OF BEING CHO CELL: 52%**

**DISPLAY FIRST CELL-LIKE OBJECT OF OBJECT No. 1 HAVING LARGE DIFFERENCE AT HIGHER POSITION**

EP 4 640 816 A1

# FIG. 21

OBJECT No. 1

95T ——

—— 95N

85

CALCULATION RESULT

CHO CELL: 62%
SIMILAR OBJECT: 28%
SCRATCH: 10%

85

CALCULATION RESULT

CHO CELL: 82%
SIMILAR OBJECT: 10%
SCRATCH: 8%

AVERAGE OF PROBABILITY OF BEING
CHO CELL: 72%

OBJECT No. 2

95T ——

—— 95N

85

CALCULATION RESULT

CHO CELL: 39%
SIMILAR OBJECT: 47%
SCRATCH: 14%

85

CALCULATION RESULT

CHO CELL: 91%
SIMILAR OBJECT: 7%
SCRATCH: 2%

AVERAGE OF PROBABILITY OF BEING
CHO CELL: 65%

DISPLAY FIRST CELL-LIKE OBJECT OF OBJECT No. 1 HAVING LARGE
AVERAGE AT HIGHER POSITION

EP 4 640 816 A1

## INTERNATIONAL SEARCH REPORT

| International application No. |
| --- |
| **PCT/JP2023/043687** |

| A. | CLASSIFICATION OF SUBJECT MATTER |
| --- | --- |

*C12M 1/34*(2006.01)i; *G06T 7/00*(2017.01)i
FI:   C12M1/34 B; G06T7/00 630

According to International Patent Classification (IPC) or to both national classification and IPC

| B. | FIELDS SEARCHED |
| --- | --- |

Minimum documentation searched (classification system followed by classification symbols)

C12M1/34; G06T7/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| X | WO 2011/016189 A1 (NIKON CORPORATION) 10 February 2011 (2011-02-10) claim 1, paragraphs [0043], [0056], fig. 6, 8 | 1-3, 6-7 |
| Y | | 4-5 |
| X | JP 2020-522251 A (MOLECULAR DEVICES, LLC) 30 July 2020 (2020-07-30) claims 1, 24-25 | 1-3, 6-7 |
| Y | | 4-5 |
| Y | JP 2015-528311 A (MOLECULAR DEVICES, LLC) 28 September 2015 (2015-09-28) claims 1, 11, paragraph [0064] | 4 |
| Y | JP 2022-509201 A (AMGEN INC.) 20 January 2022 (2022-01-20) paragraphs [0022], [0023] | 5 |
| Y | JP 2016-509844 A (PROGYNY, INC.) 04 April 2016 (2016-04-04) paragraph [0212] | 5 |

☐ Further documents are listed in the continuation of Box C.     ☑ See patent family annex.

| | |
| --- | --- |
| *   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "D"   document cited by the applicant in the international application | "X"   document of particular relevance; the claimed invention cannot be considered novel or cannot be considered to involve an inventive step when the document is taken alone |
| "E"   earlier application or patent but published on or after the international filing date | |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **09 January 2024** | **20 February 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2023/043687**

| Patent document cited in search report | | | Publication date (day/month/year) | Patent family member(s) | | | Publication date (day/month/year) |
|---|---|---|---|---|---|---|---|
| WO | 2011/016189 | A1 | 10 February 2011 | US | 2012/0134571 | A1 | |
| | | | | claim 1, paragraphs [0067], [0081], fig. 6, 8 | | | |
| | | | | EP | 2463653 | A1 | |
| JP | 2020-522251 | A | 30 July 2020 | US | 2020/0131465 | A1 | |
| | | | | claims 1, 24-25 | | | |
| | | | | WO | 2018/223142 | A1 | |
| | | | | EP | 3630939 | A1 | |
| | | | | CN | 111788297 | A | |
| JP | 2015-528311 | A | 28 September 2015 | US | 2015/0268249 | A1 | |
| | | | | claims 1, 11, paragraph [0063] | | | |
| | | | | WO | 2014/043532 | A1 | |
| | | | | EP | 2708890 | A1 | |
| | | | | KR | 10-2015-0053793 | A | |
| | | | | CN | 104838012 | A | |
| JP | 2022-509201 | A | 20 January 2022 | US | 2022/0012465 | A1 | |
| | | | | paragraphs [0028], [0029] | | | |
| | | | | WO | 2020/112723 | A1 | |
| | | | | EP | 3888004 | A1 | |
| | | | | CN | 113168519 | A | |
| | | | | KR | 10-2021-0099028 | A | |
| JP | 2016-509844 | A | 04 April 2016 | US | 2014/0247973 | A1 | |
| | | | | paragraph [0212] | | | |
| | | | | WO | 2014/134527 | A1 | |
| | | | | EP | 2961336 | A1 | |
| | | | | CN | 105407819 | A | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022509201 A